# EUROPEAN PATENT APPLICATION

(11) **EP 2 848 224 A2**
(43) Date of publication of application: **18.03.2015**
(21) Application number: 14178050.2
(22) Date of filing: 22.07.2014
(51) Int. Cl.: A61B 18/10, A61B 18/12, A61B 18/08, A61B 18/00

(54) **Tri-mode electrodes with integral temperature sensing**

(30) Priority: 25.07.2013 US 201313950687
(71) Applicant: AESCULAP AG, 78532 Tuttlingen (DE)
(72) Inventor: Kane, Mark, San Jose, CA California 95124 (US); Claude, John P., Menlo Park, CA California 94025 (US)
(74) Representative: Winter, Brandl, Fürniss, Hübner, Röss, Kaiser, Polte - Partnerschaft mbB

(57) **Abstract**

The present system relates to an apparatus for desiccating tissue. The apparatus includes an electrode assembly having a heating element, a metal electrode, and a temperature sensor. The apparatus also includes a radio frequency (RF) generator, coupled to the metal electrode, having a signal generator for generating an electrical signal, and switch for selecting between a first mode, second mode and third mode of operation. In the first mode, the electrical signal is directed to the metal electrode and applied to the tissue. In the second mode, the electrical signal is directed to the heating element such that heat produced by the heating element is applied to the tissue. In the third mode, the electrical signal is directed to the metal electrode and the heating element, for applying the electrical signal and the heat to the tissue.

## Description

### FIELD

The invention relates to electrodes for use in medical applications. More particularly, the invention relates to tri-mode electrodes with integral temperature sensing.

### BACKGROUND

In general, devices designed to desiccate tissue may use radio frequency (RF) electrical conduction through tissue to create the heat necessary to cause cellular damage. This requires that the tissue be sufficiently conductive. However, some tissue types, for example fatty tissue or mesenteric tissue, do not conduct RF energy well. To treat these poor conducting types of tissues, complex tuning of the RF generator and/or high RF voltages are required which may lead to electrical arcing and other detrimental effects. It would be advantageous to provide a device for desiccating these poor conducting tissue types without the need for complex tuning of the RF generator and/or very high RF voltages.

### SUMMARY

An embodiment of the invention provides a device for desiccating tissue. The device includes an electrode assembly having a heating element, a metal electrode, and a temperature sensor. The device also includes a radio frequency (RF) generator, coupled to the metal electrode, having a signal generator for generating an electrical signal, and switch for selecting between a first mode, second mode and third mode of operation. In the first mode, the electrical signal is directed to the metal electrode and applied to the tissue. In the second mode, the electrical signal is directed to the heating element such that heat produced by the heating element is applied to the tissue. In the third mode, the electrical signal is directed to the metal electrode and the heating element, for applying the electrical signal and the heat to the tissue.

### BRIEF DESCRIPTION OF THE DRAWINGS

Figure 1 is an electrical schematic diagram of a tri-mode electrode with integral temperature sensing according to the invention, according to an embodiment of the present invention.
Figure 2 is a perspective, partially cut-away view showing the tri-mode electrode with integral temperature sensing, according to an embodiment of the present invention.
Figure 3 is a perspective, partially cut-away view showing the tri-mode electrode in Figure 2 in contact with tissue, and connected to a generator module, according to an embodiment of the present invention.
Figure 4 is a perspective, partially cut-away view showing the tri-mode electrode split into two adjacent tri-mode electrodes, according to an embodiment of the present invention.
Figure 5 is a graph showing impedance of the tissue during the desiccation process with respect to time, according to an embodiment of the present invention.
Figure 6 is a functional flow chart showing the operation of switching between the three modes of the tri-mode electrode.

### DETAILED DESCRIPTION

Applicants have developed a method and apparatus for desiccating a wide variety of tissue types without requiring complex radio frequency (RF) tuning and/or power control. In one embodiment, electrodes are used which include a ceramic substrate formed over a heating element. The ceramic substrate may be coated and/or plated on the outside with a metal layer that forms an active electrode that comes into contact with the tissue. The ceramic substrate may also include an integral temperature sensor which may be embodied as a thermistor, thermal-couple junction or other temperature sensing device in order to measure the temperature of the metal electrode during the desiccation process.

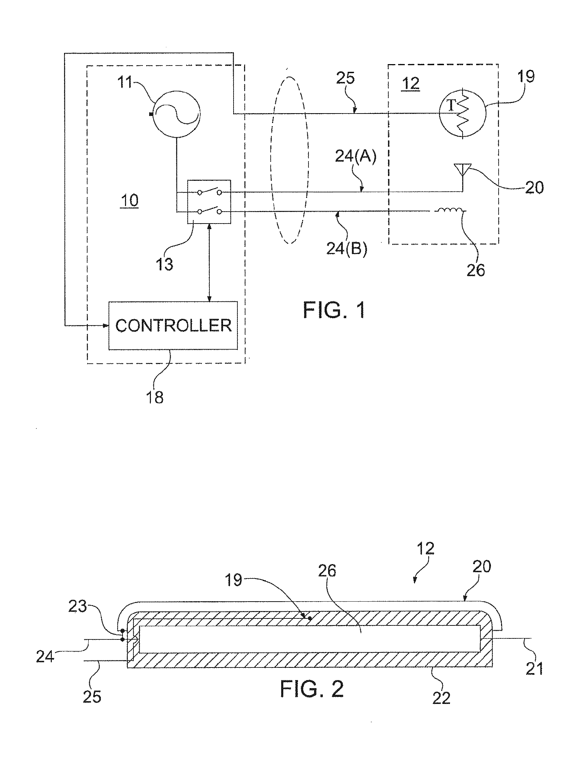

FIG. 1 is a schematic diagram of a tri-mode electrode system. Specifically, in FIG. 1, an RF generator module 10 is coupled to a tri-mode electrode 12. The RF generator module 10 may include a signal generator 11, controller 18 and a switch module 13. Signal generator 11 may generate RF signals having controllable voltages, frequencies, etc., that are used for desiccation of tissue. Switch module 13 may be utilized to independently apply the generated signal to a metal electrode 20 and heating element 26 of the tri-mode electrode 12 via electrical lines 24(A) and 24(B). It is understood that a switch module may be mechanical switches, semiconductor switches or any other type of switching mechanism that allows the power to be independently supplied to metal electrode 20 and heating element 26.

In one embodiment, generator module 10 may be controlled and monitored by controller 18. Specifically, controller 18 may monitor and/or control switch module 13. Controller 18 may also monitor and/or control signal generator 11 and a temperature sensor 19 via electrical line 25. It is noted that controller 18 may be implemented as any processor known in the art, such as microprocessor, Field Programmable Gate Array (FPGA), Application Specific Integrated Circuit (ASIC).

A structural perspective view of tri-mode electrode 12 is shown in Applicants' FIG. 2. Specifically, FIG. 2 shows a partially cut away view of the tri-mode electrode 12. Tri-mode electrode 12 may include a heating element 26 having an electrical connection 24 for receiving the RF signal from signal generator 11. Heating element 26 may also include a return electrical connection 21 which may also be connected to signal generator 11 thereby creating a current loop. In one embodiment, heating element 26 may be a resistive heating element that produces heat in response to an applied RF signal.

Tri-mode electrode 12 may also include a non-conductive substrate 22 covering a portion or completely enveloping heating element 26. This non-conductive substrate ensures that the electrical current flowing through the heating element does not pass to metal electrode 20 or into the tissue of the patient during the desiccation process.

In FIG. 2, tri-mode electrode 12 may also include a metal layer 20 (i.e. metal electrode) that may be implemented as a metal coating on the non-conductive substrate or a metal plate bonded to the non-conductive substrate. Metal layer 20 is the portion of tri-mode electrode 12 that comes into contact with the tissue of the patient during the desiccation process.

Also shown in FIG. 2 is temperature sensor 19 that is integral to the non-conductive substrate 22 and placed in between heating element 26 and metal layer 20. Since temperature sensor 19 is located in between heating element 26 and metal layer 20, heating element 19 may be able to accurately determine the temperature of metal electrode 20 which is applied to the tissue of the patient during the desiccation process. The temperature detected by temperature sensor 19 may also be returned to controller 18 via signal line 25 for analysis purposes.

Tri-mode electrode 12 in FIG. 2 also shows wire 24 connected to heating element 26 and metal electrode 20 via a common connector 23. This allows the RF source produced by signal generator 11 to be applied to both heating element 26 and metal electrode 20 simultaneously through one wire. In this example (with common connector 23), switch module 13 could be connected to return connection 21 of heating element 26 (not shown) in order to control heating element 26 and metal electrode 20 separately.

It is also contemplated in FIG. 1 that common connector 23 may not be included. Specifically, two independent wires 24(A) and 24(B) as shown in FIG. 1, may be connected to metal electrode 20, and heating element 26 respectively. Each of these wires 24(A) and 24(B) would then be connected to switch module 13 thereby controlling the supply of the RF signal independently to both elements.

Integrated temperature sensor 19 of tri-mode electrode 12 may be embodied by a current sensing resistor, a current transformer, or any other relevant current sensor. It is also noted that, in one example, the tri-mode electrode 12 may not include an integrated temperature sensor. An external temperature sensor may be implemented. For example, an optical sensor may monitor the surface temperature of metal electrode 20 from another location (e.g., at another location on the overall electrode assembly).

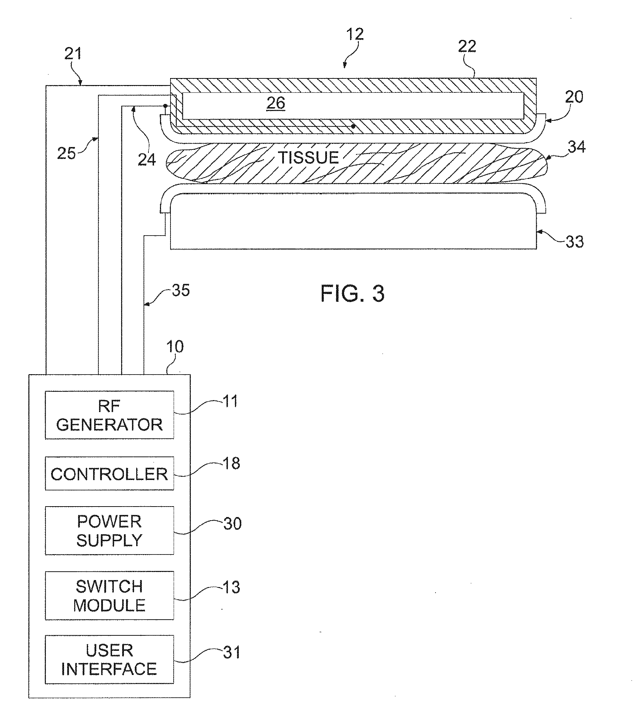

As described above, metal electrode 20 comes into contact with the tissue of the patient. In one embodiment (as shown in FIG. 3), tri-mode electrode 12 may be part of a larger overall electrode assembly that includes a return electrode 33. In general, return electrode 33 may be similar in overall configuration as tri-mode electrode 12 (i.e. return electrode 33 may also be a tri-mode electrode). In other embodiments, return electrode 33 may not have any heating and/or RF producing capabilities (i.e. return electrode 33 would return the RF electrical current to the source).

In the example shown in FIG. 3, tri-mode electrode 12 and return electrode 33 are configured as a clamping electrode assembly. More specifically, tissue 34 to be desiccated is clamped in between tri-mode electrode 12 and return electrode 33. The RF generator module 10, may similarly include signal generator 11, controller 18, switch module 13, power supply 30 and user interface 31.

Tri-mode electrode 12 may be electrically connected to module 10 via electrical lines 21, 24 and 25. Return electrode 33 may also be electrically connected to module 10 via return electrical line 35. If return electrode 33 is also an active electrode, then return electrode 33 may have additional electrical connections to the heating element and a temperature sensing sensor if available (not shown).

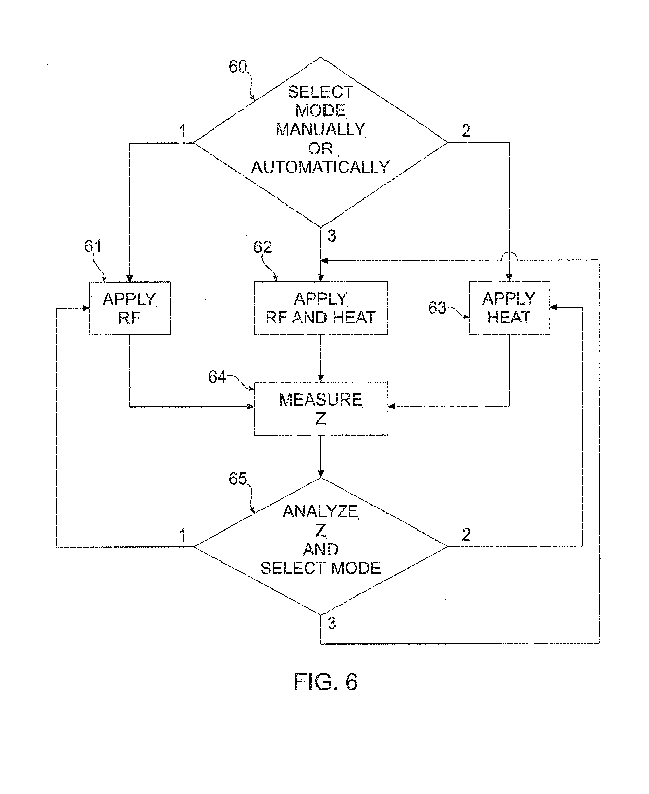

In general, during operation, power supply 30 would supply power to the various components of module 10 including signal generator 11 which generates the RF signal applied to the electrodes 12 and 33. Controller 18 would control switch module 13, signal generator 11 and user interface 31 in order to apply the proper RF treatment and/or heat treatment to tissue 34. User interface 31 may allow a user (e.g., a surgeon) to manually control the RF treatment and/or heat treatment being applied to tissue 34. More details of the overall control of switching between the various modes will be described with respect to FIG. 6.

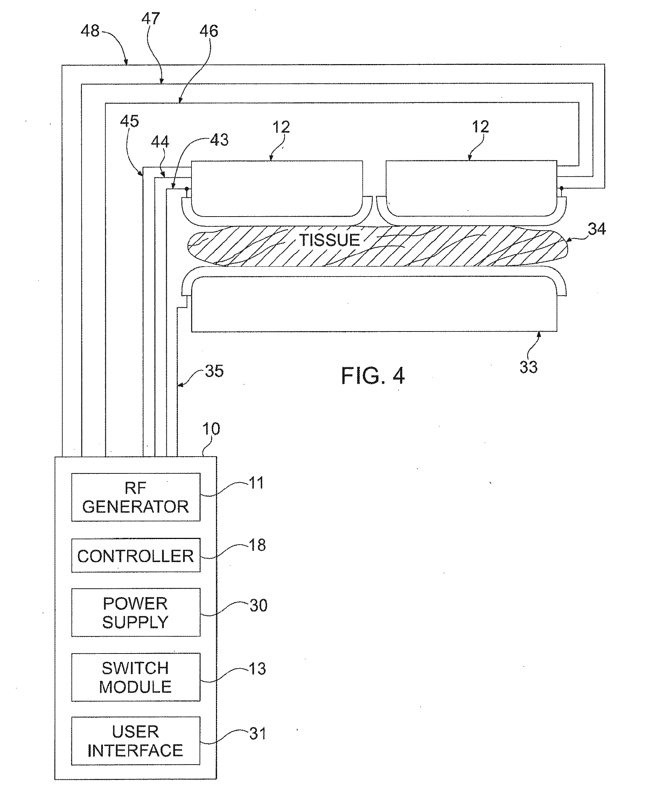

FIG. 4 shows another electrode system featuring two smaller tri-mode electrodes 12. Tri-mode electrodes 12, which are similar to tri-mode electrode 12 in FIG. 3, may be electrically connected to a module 10 via electrical lines 43, 44, 45, 46, 47 and 48. More specifically, the RF signal may be supplied to electrodes 12 via lines 43 and 48, respectively. The electrical signal generated by the temperature sensor may be fed back to module 10 via electrical lines 44 and 47, respectively. Furthermore, the return current from the heating elements of the electrodes may be returned to module 10 via electrical lines 45 and 46, respectively.

The two smaller tri-mode electrodes 12 may be controlled independently of one another to apply RF treatment and/or heat treatment to different portions of tissue 34. In general, controller 18 may control both of the tri-mode electrodes 12 to apply RF and/or heat to tissue 34 simultaneously, or at different times, or in a particular sequence. The return current from tri-mode electrodes 12 may be fed back to module 10 via return electrode 33 and return electrical line 35.

As already described, the user interface 31 may be utilized by the surgeon in order to manually control the application of RF energy and/or heat to tissue 34 of the patient. However, this control may also be performed automatically by controller 18. More specifically, the application of RF energy and/or the application of heat may be automatically controlled based on the measured impedance of tissue 34 during the desiccation process. Furthermore, the voltage, current, frequency, and phase of the RF signal may also be automatically and/or manually selected based on the impedance of tissue 34.

In one example, the current and voltage being applied to the tri-mode electrode may be monitored by controller 18, thereby allowing the impedance of the tissue to be computed at any point in time during the desiccation process. Controller 18 may then compare the instantaneous value of the impedance, a statistical value of the impedance or the gradient of the impedance to various thresholds in order to determine when to switch between applying RF energy (first mode), applying heat (second mode) or applying both RF energy and heat (third mode).

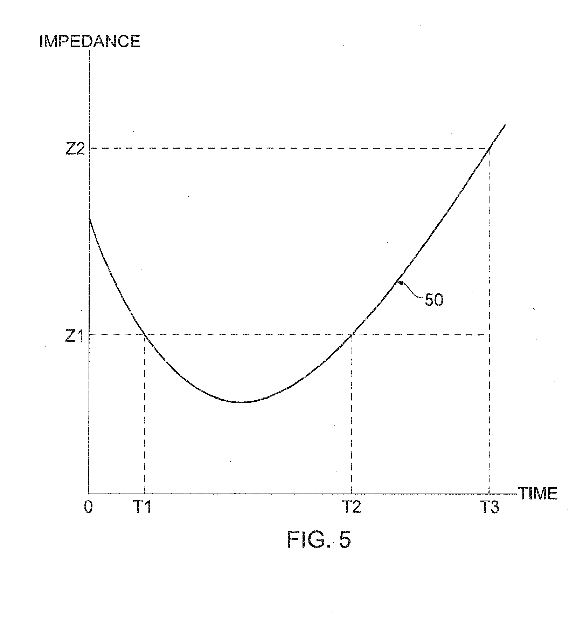

For example, Fig. 5 shows plot 50 of the impedance of tissue 34 versus time during the desiccation process. Assuming that RF energy is being applied to the tissue (first mode) at time 0, the impedance of the tissue may begin to decrease. Optional heat may also be applied simultaneously (third mode) to reduce the time T1 to reach impedance threshold Z1.

Once the impedance reaches threshold Z1, controller 18 may determine that the impedance is too low which may lead to high current in order to obtain the power required for desiccation. In this example, heat may be applied simultaneously (third mode) to dehydrate the tissue (i.e. increase the impedance) and reduce the time T2 to reach impedance threshold Z1. Once the impedance of the tissue is increased above threshold Z1, the RF and heat may continue to be applied simultaneously (third mode). If the voltage, which is required to reach the necessary RF energy for desiccation becomes too high (e.g. at time T3), the RF can be switched off, and only heat is applied to the tissue for the final part of the desiccation process (second mode).

FIG. 5 is only meant to be an example of one possibility for controlling the application of RF energy and heat to the tissue based on the impedance of the tissue. It is contemplated that the controller 18 may control the application of the RF energy and/or the heat using various algorithms that depend on instantaneous values of the impedance, statistical values of the impedance, or gradients of impedance with respect to time.

Overall, controller 18 is able to control tri-mode electrodes 12 by selecting between three different modes (a first mode where only RF energy is applied, a second mode where only heat applied and a third mode where both RF energy and heat is applied to the tissue). Controller 18 may then measure and/or compute the impedance of tissue 34, analyze the impedance, and then either continue to maintain the selected mode or switch to a different mode depending on which best suits the tissue. An example of this control is shown in the flow chart of Applicants' FIG. 6.

As shown in Applicants' FIG. 6, (step 60) the initial mode may be selected either manually by the surgeon operating tri-mode electrode assembly 12 or automatically by controller 18. Automatic selection by controller 18 may be performed in a variety of ways. Specifically, controller 18 may send a test current through tissue 34 to determine the initial impedance prior to the desiccation process. This initial impedance may then be utilized to select either the first, second or third mode. Alternatively, controller 18 may also utilize known parameters of the patient that have been input to module 10 (i.e., patient's age, sex, blood type, body fat percentage, etc.) either independently or in conjunction with an initially measured impedance in order to select either the first, second or third mode.

In general, once an initial mode is selected, then either the RF is applied (step 61), the heat is applied (step 63) or both the RF and heat are applied (step 62). Regardless of the mode that is selected, the controller 18 may then measure the impedance of the tissue as it is being desiccated (step 64). This measured impedance is then analyzed (step 65). Specifically, the impedance may be analyzed and compared to impedance thresholds, gradients, and other statistical values in order to allow controller 18 to make an intelligent decision on which mode is more appropriate for desiccating tissue 34 at any given point in time. Depending on this analysis, controller 18 may then select either the first mode, second mode or third mode, at which point the overall flow process would be repeated.

Although FIG. 6 shows the automatic control of the operating modes of tri-mode electrode 12, it is also noted that the user interface will still allow for manual intervention by the doctor. It is also noted that the surgeon may also control the parameters of the automatic algorithm. For example, the surgeon may manually set the impedance thresholds, gradients or statistical measures that are being utilized by controller 18 during the automatic control process. This allows the surgeon to have complete control over both the manual and automatic aspects of the overall system.

Although the invention is illustrated and described herein with reference to specific embodiments, the invention is not intended to be limited to the details shown. Rather, various modifications may be made in the details within the scope and range of equivalents of the claims and without departing from the invention.

## Claims

1. An apparatus for desiccating tissue, said apparatus comprising:
an electrode assembly including:
a heating element,
a metal electrode, and
a temperature sensor located in proximity to said metal electrode; and
a radio frequency (RF) generator, coupled to said metal electrode, including:
a signal generator for generating an electrical signal for application to at least one of said heating element and said metal electrode, and
a switch module for selecting between:
a first mode for conduction, in which said electrical signal is directed to said metal electrode and applied to said tissue,
a second mode for heating, in which said electrical signal is directed to said heating element and heat produced by said heating element is applied to said tissue, and said heat of metal electrode is monitored via said temperature sensor, and
a third mode, in which said electrical signal is directed to said metal electrode and said heating element, for applying said electrical signal to said tissue, and applying said heat to said tissue.

2. The apparatus according to any of the preceding claims, including:
a user interface for manually selecting between the first mode, second mode and third mode, and for manually controlling parameters of the electrical signal.

3. The apparatus according to any of the preceding claims, including:
a common electrical connection between the heating element and the metal electrode such that they receive the electrical signal simultaneously.

4. The apparatus according to any of the preceding claims, including:
a metal return electrode positioned on an opposite side of the tissue as the metal electrode, such that the electrical signal passes from the metal electrode, through the tissue and into the return electrode.

5. The apparatus according to any of the preceding claims, including:
another electrode assembly coupled to the RF generator and positioned adjacent to the electrode assembly with respect to the tissue,
wherein the electrode assembly and the other electrode assembly apply the electrical signal to adjacent portions of the tissue independently of each other.

6. The apparatus according to any of the preceding claims, including:
a controller coupled to the switch module and configured to automatically control the selection between the first mode, second mode and third mode based on a measured impedance of the tissue.

7. The apparatus of claim 6, wherein:
the controller controls the selection of the mode based on at least one of an impedance threshold, or a predetermined gradient of the impedance.

8. A method for desiccating tissue using an apparatus for desiccating tissue, said apparatus including an electrode assembly having a metal electrode and a heating element, and a radio frequency (RF) generator for generating an RF signal, the method comprising:
selecting, by the RF generator, one of:
a first mode for conduction, in which said RF signal is directed to said metal electrode and applied to said tissue,
a second mode for heating, in which said RF signal is directed to said heating element and heat produced by said heating element is applied to said tissue, and
a third mode, in which said RF signal is directed to said metal electrode and said heating element simultaneously, such that said RF signal and said heat are applied to said tissue at the same time.

9. The method of claim 8, including the steps of:
manually selecting, by a user interface, one of the first mode, second mode and third mode; and
manually setting, by the user interface, parameters of the RF signal.

10. The method of claim 8, including the step of:
applying the RF signal, by another electrode assembly, to another portion of the tissue independent of the electrode assembly.

11. The method of claim 8, including the step of:
automatically selecting, by a controller, one of the first mode, second mode and third mode based on a measured impedance of the tissue.

12. The method of claim 11, wherein the controller selects one of the first mode, second mode and third mode based on a comparison of the measured impedance to a threshold impedance.

13. The method of claim 11, wherein the controller selects one of the first mode, second mode and third mode based on a comparison of a gradient of the measured impedance with a predetermined gradient.

14. The method of claim 11, wherein the controller applies the RF signal to the tissue, and applies the heat to the tissue in a periodic manner.

15. An electrode assembly for desiccating tissue, said electrode assembly comprising:
a heating element for applying heat to said tissue in response to receiving an electrical signal;
a substrate covering said heating element;
a metal electrode on a portion of said substrate for applying said electrical signal to said tissue in response to receiving said electrical signal; and
a temperature sensor integrated within said substrate for detecting a temperature of said metal electrode.

16. The apparatus of claim 15, wherein
the temperature sensor is located between the heating element and the metal electrode.

17. The apparatus according to any of claims 15 to 16, wherein
the substrate is non-conductive, and completely envelopes the heating element.

18. The apparatus according to any of claims 15 to 17, including:
another electrode assembly positioned adjacent to the electrode assembly and contacting adjacent tissue,
wherein the two electrode assemblies apply the heat and the electrical signal to the tissue and the adjacent tissue independently of each other.

19. The apparatus according to any of claims 15 to 18, including:
a common electrical connection between the heating element and the metal electrode such that the electrical signal is supplied to the heating element and the metal electrode simultaneously.

20. The apparatus according to any of claims 15 to 19, including:
a metal return electrode positioned on an opposite side of the tissue as the metal electrode to form a clamp for clamping the tissue,
wherein the electrical signal passes from the metal electrode, through the clamped tissue and into the return electrode.
